# EUROPEAN PATENT APPLICATION

(11) **EP 4 186 533 A1**
(43) Date of publication of application: **31.05.2023**
(21) Application number: 20963589.5
(22) Date of filing: 30.11.2020
(51) Int. Cl.: A61L 15/40

(54) **BURN REPAIRING MATERIAL**

(71) Applicant: National University Corporation University Of Toyama, Toyama-shi, Toyama 930-8555 (JP); Sakura Seiki Co., Ltd, Chikuma-shi, Nagano 387-0015 (JP)
(72) Inventor: OBA Jiro, Toyama-shi, Toyama 930-0194 (JP); YOSHIDA Toshiko, Toyama-shi, Toyama 930-0194 (JP); OKABE Motonori, Toyama-shi, Toyama 930-0194 (JP); SOKO Chika, Toyama-shi, Toyama 930-0194 (JP); ARAKAWA Masahiko, Chikuma-shi, Nagano 387-0015 (JP)
(74) Representative: Mewburn Ellis LLP
(86) International application number: PCT/JP2020/044438
(87) International publication number: WO 2022/113325

(57) **Abstract**

Raw amniotic membrane placed inside a processing tank (10) is continuously heated by a far-infrared heater (14) provided inside the processing tank (10). A decompression operation, where the interior of the processing tank (10) is placed in a decompressed state, and irradiation of the raw amniotic membrane with microwaves from a microwave irradiating device (30) provided inside the processing tank (10) to apply energy to the water molecules present inside the amniotic membrane and dry the amniotic membrane during a pressure recovery operation that slightly raises the pressure inside the depressurized processing tank (10) toward atmospheric pressure are performed. After removal of a necrotic layer and the like from a patient with a severe burn, amniotic membrane, which has been dried by repeating the above multiple times so as to preserve its cell and tissue structure, is used as a covering material and scaffold on a wound. By doing so, cytokines and physiologically active substances secreted from infiltrating cells are retained, and favorable granulation formation, including neovascularization and fibroblast proliferation, is promoted, which makes it possible to increase the patient's survival rate and achieve therapeutic effects (that is, eliminate the risk of keloids).

## Description

### Technical Field

The present invention relates to a restoration material used as a burn covering material and/or a regenerative scaffold for burns for use in the treatment of burns, and in particular serious burns.

### Background Art

The severity of a burn is determined by the area and depth. Depth is classified into first to third degrees based on the color tone of the surface. A second-degree burn is a burn that reaches the dermis, and a third-degree burn is a state where the skin is entirely damaged.

When the body suffers second-degree burns to 30% or more of the body surface area or third-degree burns to 10% or more of the body surface area, intensive care at an emergency center becomes necessary.

Third degree burns in the region subjected to emergency surgery will generally have thick necrotic tissue and will be subjected to surgical treatment, which prevents the application of a general-purpose wound dressing. If there is the risk of local infection occurring over time, eschar and necrotic tissue should be removed as soon as possible, and skin grafting should be performed after observing the formation of granulation tissue from the surrounding area. However, due to insufficient wound bed preparation (that is, favorable granulation), which is indispensable when grafting is performed, there is often the problem of deterioration in the patient's condition and even death due to poor regeneration (that is, difficulty in achieving engraftment of transplanted skin).

According to the "Clinical Practice Guidelines for Management of Burn Care" [Revised 2nd Edition], 2015" published by the Japanese Society for Burn Injuries, most reports about wound dressings used on burns relate to second degree burns. There is no wound dressing that has been actively used on third degree burns. Wound dressings used for burns are roughly divided into foam materials, fiber materials, and colloidal materials, which are selectively used as appropriate according to shape and ability to absorb exudate.

### Citation List

### Patent Literature

Japanese Laid-open Patent Publication No. 2007-54015

### Non Patent Literature

Non Patent Literature 1: Gruss, J. S.& Jirsch, D. W: "Human amniotic membrane: A versatile wound dressing". Can. Med. Assoc. J. 118, 1237-1246 (1978).
Non Patent Literature 2: Okabe, M. et al. "Hyperdry human amniotic membrane is useful material for tissue engineering: Physical, morphological properties, and safety as the new biological material". J. Biomed. Mater. Res. - Part A 102, 862-870 (2014).

### Summary of Invention

### Technical Problem

Until now, no treatment method for third-degree burns has had an effect of preventing infection and promoting favorable granulation, and at present there is no choice but to rely on the vitality and immunity of the patient.

Although it is necessary to grow granulation tissue at an early stage, there is no effective material to achieve this. Forming favorable granulation at an early stage and performing a skin graft at an early stage without causing infection are effective in prolonging the life of a patient with serious burns.

### Solution to Problem

Amniotic membrane is a tough biological membrane composed of collagen and elastic fibers, and raw amniotic membrane has been reported as a useful covering material for trauma and burns (see Non-Patent Document 1). However, raw amniotic membrane has not been available immediately when needed and has been complicated to store and handle, which has limited its use in actual clinical practice. On the other hand, dry amniotic membrane (or "hyper-dry huma amniotic membrane": hereinafter, "HD-AM") produced by a specific drying process has also been announced (see Patent Literature 1 and Non Patent Literature 2).

The present inventors created burn model animals with first to third degree burns, and histologically, immunochemically, and molecularly confirmed that HD-AM promotes granulation growth at dermal defects or subcutaneous tissue defects of the burn model animals. In other words, the present inventors completed the present invention by clarifying that HD-AM functions as a scaffold for cells, induces growth factors and cell migration chemokines, and regulates differentiation to M2 macrophages to promote local anti-inflammation and tissue regeneration, thereby contributing to tissue regeneration.

The present invention will be described in detail below.

A restoration material for burns according to the present invention is a restoration material to be used in treatment of burns as a covering material for burns and/or a regenerative scaffold for burns, the restoration material being a dry amniotic membrane produced by performing a drying process on raw amniotic membrane that surrounds a fetus of an animal, including a human, wherein the dry amniotic membrane has been dehydrated and dried so as to enable storage in an aseptic dry atmosphere, and amniotic membrane produced by rehydrating the dry amniotic membrane by immersion in water or a buffer retains epithelial cells, basement membrane, and connective tissue constituting the raw amniotic membrane.

By using this configuration, when the restoration material is used as a covering material and/or a scaffold for regeneration of a dermal defect or subcutaneous tissue, especially for a patient with a severe burn, secretion of physiologically active substances, such as inflammatory cytokines and growth factors, is promoted at the wound site of the severe burn. The inflammatory cytokines protect against infection by foreign substances from the outside, and wound healing of the severe burn is additionally promoted through active effects, such as promotion of secretion of VEGF and TGF-b.

The restoration material may be a covering material and/or a scaffold for a treatment process of severe burns that are second-degree burns and/or third-degree burns.

Use of a restoration material for burns according to the present invention is use of a restoration material for burns to be used in treatment of burns as a covering material for burns and/or a regenerative scaffold for burns, the restoration material being a dry amniotic membrane produced by performing a drying process on raw amniotic membrane that surrounds a fetus of an animal, including a human, wherein the dry amniotic membrane has been dehydrated and dried so as to enable storage in an aseptic dry atmosphere, and amniotic membrane produced by rehydrating the dry amniotic membrane by immersion in water or a buffer retains epithelial cells, basement membrane, and connective tissue constituting the raw amniotic membrane.

This use may be use as a covering material and/or a scaffold for a treatment process of severe burns that are second-degree burns and/or third-degree burns.

### Advantageous Effects of Invention

By applying the HD amniotic membrane, cell infiltration becomes active in an early stage post-injury, and by holding infiltrating cells as a scaffold, it becomes possible to promote the secretion of physiologically active substances, such as inflammatory cytokines and growth factors, at the wound site. It was established that inflammatory cytokines protect against infection by foreign substances from outside, which promotes the secretion of VEGF and TGF-b, and promotes neovascularization and favorable granulation formation. On the other hand, secretion of IL-10, a cytokine that suppresses inflammation, was stimulated, and CD163-positive M2 cells had significantly proliferated by POD7.

### Brief Description of Drawings

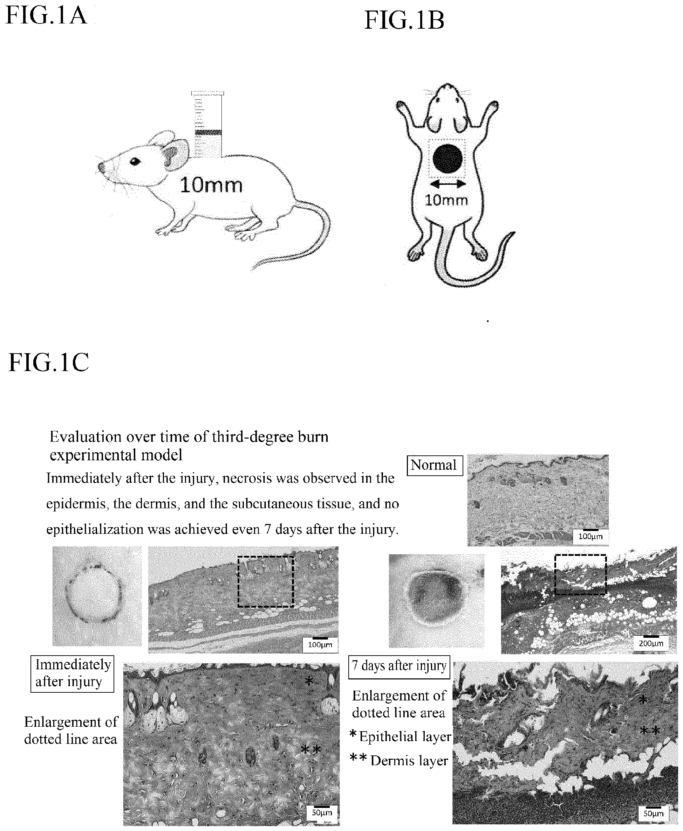

FIGS. 1A to C depict an experimental mouse model and an application of HD-AM (in the drawings, "adipose tissue" indicates adipose tissue, tissue 1 and 2 = good granulation tissue). FIG. 1A is a schematic diagram of an experimental mouse (creation of a burn site). After a mouse has been anesthetized and placed in the prone position, the four limbs are immobilized. The skin on the back was shaved and hair was removed with depilatory cream. An area of 10 mm on the back was exposed to hot water at 90°C for 10 seconds to form a third-degree burn wound with a size of ϕ = 10 mm in the center of the back. FIG. 1B is a schematic diagram of an experimental mouse (initiation of treatment by removing burned part).
Immediately after the burn, a full thickness of skin of the same site was excised to create an open wound. FIG. 1C is a diagram in which time-based evaluation of this third-degree burn experimental model was observed. An area with a diameter of 10 mm was exposed to hot water at 90°C for 10 seconds, and the depth of the burn was observed. Immediately after the injury, necrosis was observed in the epidermis, the dermis, and the subcutaneous tissue, and no epithelialization was observed even 7 days after the injury. From these results, it was judged that this experimental model is appropriate as a third-degree burn model, resulting in its use as a third-degree burn model for examining the effects, such as granulation formation, of burn treatments.

FIGS. 2A to C are diagrams useful in explaining application of HD-AM. FIG. 2A schematically depicts a mouse where HD-AM has been used at a wound. FIG. 2B schematically depicts a mouse (control) where HD-AM is not used at a wound. FIG. 2C schematically depicts placement of HD-AM on a wound area.

FIG. 3 indicates primers used in quantitative reverse transcription polymerase chain reaction (qRT-PCR).

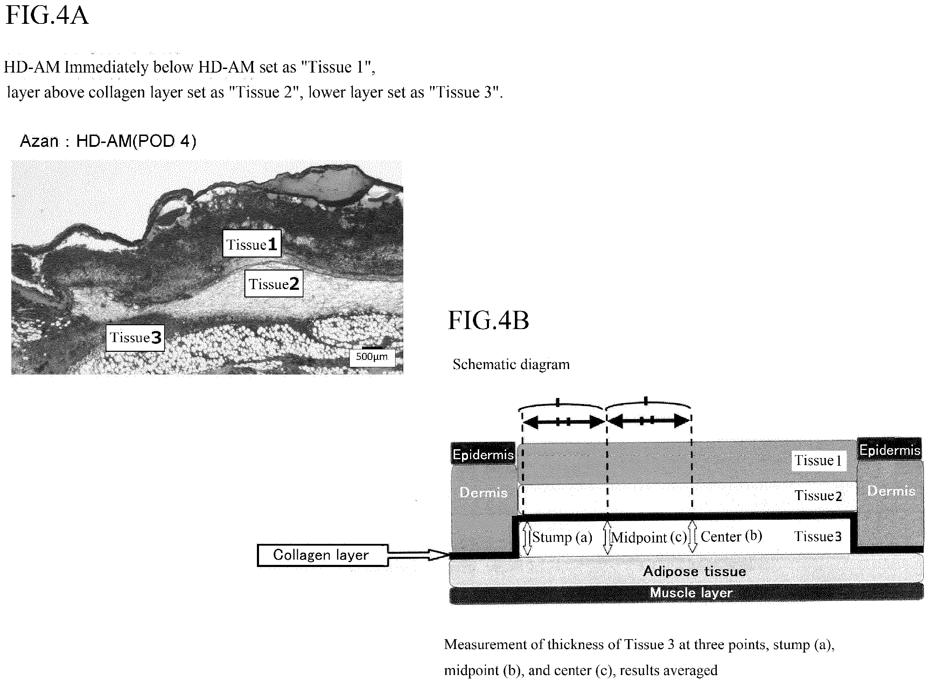

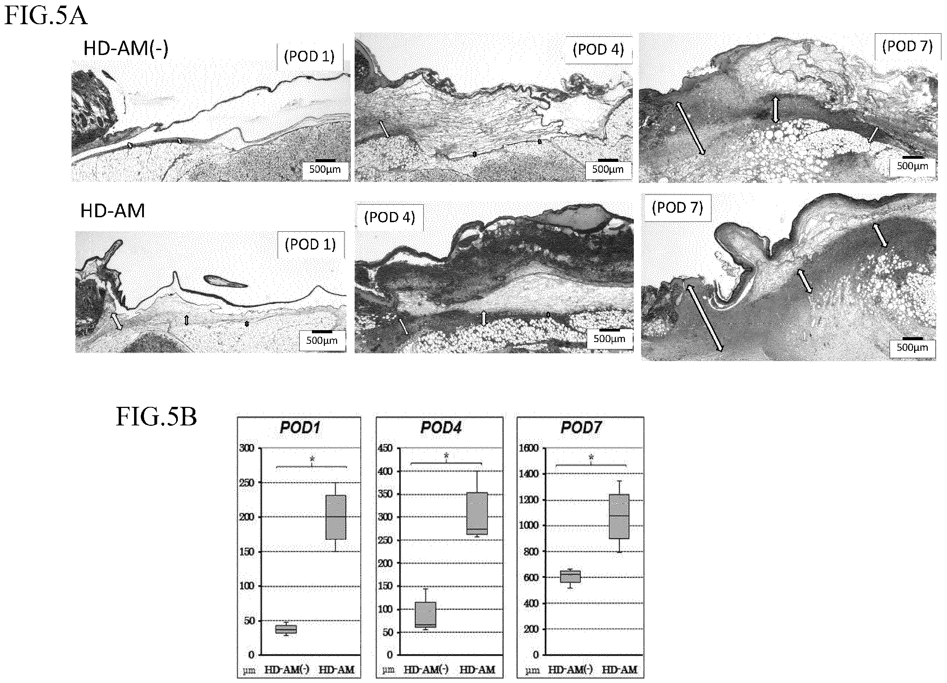

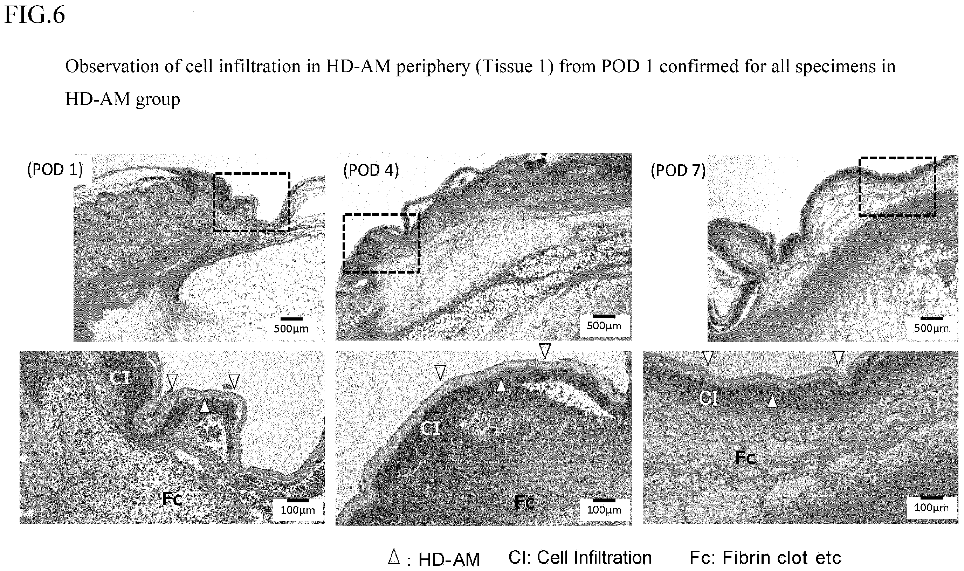

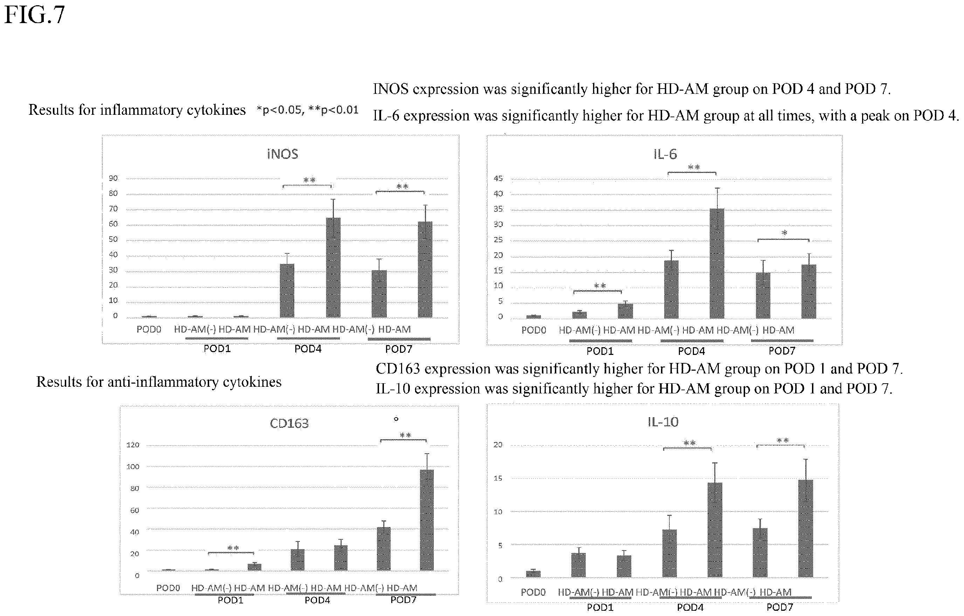

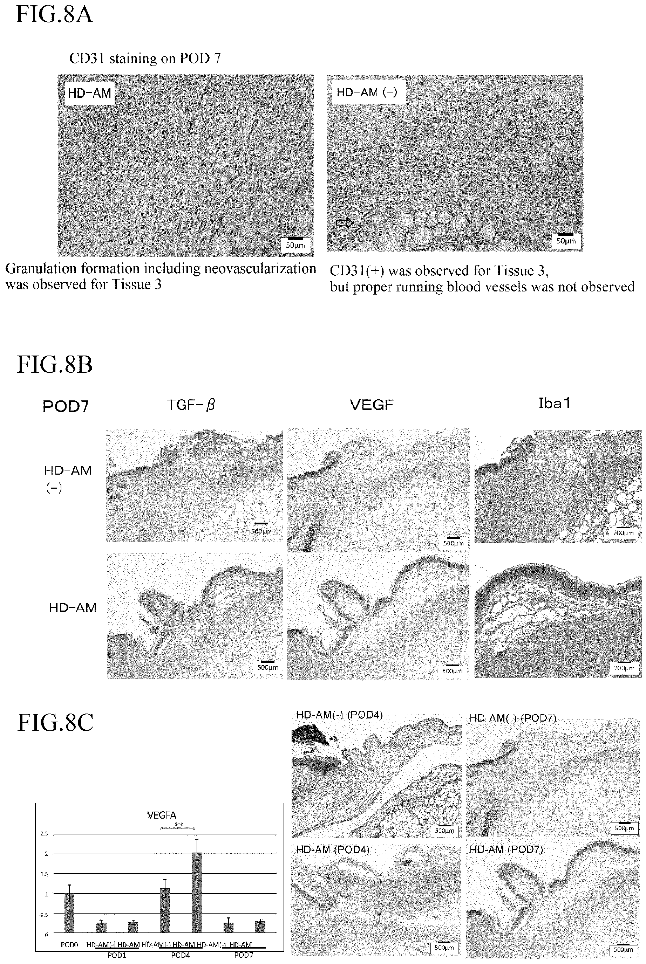

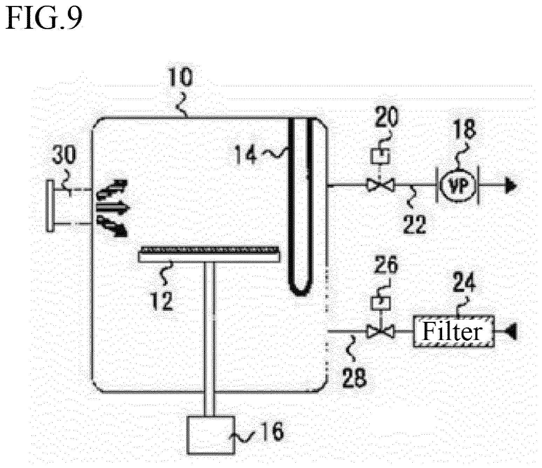

FIGS. 4A and B are schematic diagrams of a method of measuring the thickness of granulation tissue. FIG. 4A is a diagram depicting observation over time of tissue that has been stained by azan staining, which selectively stains connective tissue, following a burn injury. FIG. 4B is a schematic diagram depicting measurement of the thickness of connective tissue as part of treatment after a burn injury. FIGS. 5A and B are representative photomicrographs of post-injury treatment. FIG. 5A is representative photomicrographs of an HD-AM (-) group on days 1, 4, and 7 after treatment (POD 1,4,7) and an HD-AM group on days 1, 4, and 7 after treatment (POD 1,4,7). The thickness of the granulation tissue is indicated by yellow arrows. FIG. 5B is a graph comparing the measured average thickness of granulation tissue. The HD-AM group showed significantly thicker granulation tissue than the HD-AM (-) group on all of days 1, 4, and 7 after treatment (POD 1, 4, and 7) (n = 5, ^{∗} p < 0.05). FIG. 6 is a diagram explaining cell infiltration into HD-AM. Observation of cell infiltration into HD-AM used H-E staining (x50, x100). When the amniotic membrane placed at the wound was observed histologically, higher inflammatory cell infiltration was confirmed for the HD-AM group at a lower part of the amniotic membrane. This is believed to indicate the amniotic membrane functioning as a scaffold. FIG. 7 is a graph in which various growth factors, cell migration chemokines, and anti-inflammatory and inflammatory cytokines were measured using quantitative RT-PCR. Measurement of anti-inflammatory and inflammatory cytokines. For the HD-AM group, an elevated expression of inflammatory cytokine mRNA is observed, followed by abundant expression of anti-inflammatory cytokine mRNA. This suggests that the wound healing process was clearly and smoothly promoted. FIGS. 8A to C are photomicrographs where immunohistochemical staining was performed. FIG. 8A is a comparison of CD31 staining on POD 7. Greater fibroblast proliferation and neovascularization were observed for the HD-AM group on POD 7. These newly formed blood vessels and fibers were elongated in a direction toward tissue 1 within a tissue 3 region. FIG. 8B is a comparison of cell infiltration on POD 7. At tissue 1, infiltrated cells had engrafted the HD-AM even on POD 7, with such cells being positive for TGF-b, VEGF, and Iba-1. FIG. 8C depicts an observation and comparison of VEGF secretion sites (cells) on POD 4 and POD 7. FIG. 9 depicts a drying device for producing HD amniotic membrane.

### Description of Embodiments

### Overview of Embodiments

A dried amniotic membrane manufactured by a specified drying process (called "hyperdrying") is, for example, the dried amniotic membrane described in Patent Literature 1. That is, raw amniotic membrane placed in a processing tank is continuously heated by a far-infrared heater provided inside the processing tank, and a depressurization operation, in which the inside of the processing tank is placed in a depressurized state, and irradiation of the raw amniotic membrane with microwaves from a microwave irradiating device provided inside the processing tank to apply energy to water molecules present inside the amniotic membrane and cause drying during a pressure recovery operation that slightly raises the pressure inside the depressurized processing tank toward atmospheric pressure were performed. In the dried amniotic membrane (HD-AM) manufactured by repeating the above process a plurality of times, the amniotic membrane cells themselves are inactivated but the cell and tissue structures are retained.

Third degree burns in the region of emergency surgery fundamentally have thick necrotic tissue and are indicated for surgical treatment, which prevents the application of a general-purpose wound dressing. If there is the risk of local infection occurring over time, eschar and necrotic tissue should be removed as soon as possible, and skin grafting should be performed after observing the formation of granulation tissue from the surrounding area. However, due to insufficient wound bed preparation (that is, favorable granulation), which is indispensable when grafting is performed, there is often the problem of deterioration in the patient's condition and even death due to poor regeneration (difficulty in achieving engraftment of the transplanted skin).

In the present embodiment, HD-AM is placed as a covering material and a scaffold. Placing HD-AM as a covering material and a scaffold on the wound part of a severe burn promotes protection against infection and neovascularization through a transient elevation of inflammatory cytokines and VEGF, which then changes to a decrease in the inflammatory cytokines and VEGF and an increase in anti-inflammatory cytokines, which promotes active effects such as high-quality granulation.

In the present embodiment, when HD-AM is used as a covering material and a scaffold, as one example, the HD-AM may be formed into an appropriate shape with scissors or the like in keeping with a wound site of a burn, severe burn, or the like where a general-purpose wound covering material cannot be used, and a drainage hole may be fabricated.

### Creation of Burn Wounds in Animal Models

As depicted in FIG. 1, mice were anesthetized, placed in the prone position, and then had their four limbs immobilized. The skin on the back was shaved and the hair was removed with depilatory cream. A 10 mm area was exposed to boiling water at 90°C for 10 seconds using a tube placed on the back (see FIG. 1A) to form a third-degree burn wound in the center of the back with a size of ϕ = 10 mm. The full-thickness of skin at the same site immediately after the burn was excised to make an open wound (see FIG. 1B). In this method, the depth of the burn can be adjusted by changing the temperature of the introduced hot water and the exposure time of the hot water, and the area of the burn can also be changed by changing the diameter of the tube in use. By doing so, it is possible to easily and stably create burn models as needed, using inexpensive materials for burns that were previously created using expensive equipment.

Note that the handling of laboratory animals was approved by the Institutional Animal Care and Use Committee of the University of Toyama in accordance with the guidelines of the National Institutes of Health. The experiments were conducted according to the guidelines of the Institutional Animal Care and Use Committee of the University of Toyama.

### Application of HD-AM

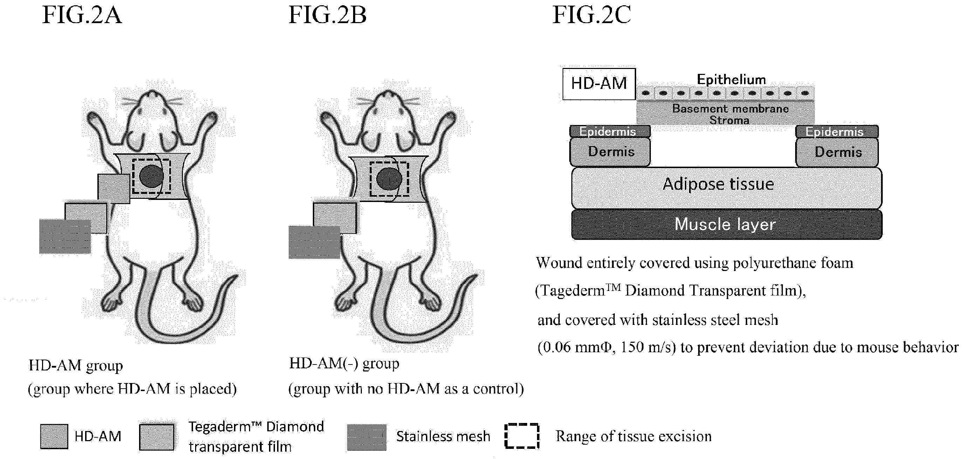

Human amniotic membrane is mainly composed of three layers, a monolayer epithelial layer (hereinafter "epithelium"), a thin basement membrane (hereinafter "basement membrane"), and an avascular stromal layer (hereinafter "stroma") (see the HD-AM part of FIG. 2C). In the experiment, the HD-AM was cut into 15 × 15 mm sizes and two groups (see FIGS. 2A and 2B) were formed composed of a group with the epithelium facing up (hereinafter "HD-AM group"), and a group where no HD-AM was placed as a control (hereinafter "HD-AM(-) group"). The burn wound (the wall of exposed bowel) was entirely covered with a polyurethane foam covering material (Tegaderm ^{™} Diamond transparent film (R), made by 3M Deutschland GmbH Health Care Business, Germany).

Each of the groups ("HD-AM(-)" and "HD-AM") contained five mice which were evaluated 1 day post operation (POD 1), 4 days post operation (POD 4), and 7 days post operation (POD 7), meaning a total of thirty animals were used. The wounds were covered with stainless steel mesh (0.06 mmΦ, 150 m/s) to prevent deviation of the HD-AM and/or wound covering due to mouse behavior.

### Histological and Immunohistochemical Staining

To perform histological observation, the wounded part produced by the burn was collected from each mouse on POD 1, POD 4, and POD 7 and embedded in paraffin. Sections sliced from the paraffin block were stained with hematoxylin-eosin (H&E) and Azan. In addition, immunohistochemical staining was also performed for CD163, Iba1, TGFβ-1 (transforming growth factor beta-1), VEGF (vascular endothelial growth factor), CD31, and αSMA. Stained tissue was imaged using a Leica DMRBE microscope (Leica, Wetzlar, Germany) and a DP73 system (Olympus, Tokyo, Japan).

### Measurement of Granulation Tissue (Regenerated Part) at the Treatment Location of Burn

Azan staining makes it easy to distinguish between collagen fibers and fibrin for observation of granulation tissue. The thickness of granulation tissue containing collagen fibers was measured using the Olympus CellSens imaging program (version 1.7; Olympus, Tokyo, Japan). On the 4th day after treatment (POD 4), regardless of whether HD amniotic member was applied, the appearance of a layer of collagen membrane between the subcutaneous tissue and muscle layer of the surrounding normal tissue was confirmed. Since movement of infiltrating cells at the edge and granulation formation were observed for this membrane, regions were determined as depicted in FIG. 4A. Changes over time in these regions were observed, measurement was performed at three points a, b, and c where the measurement sites were an epithelium defect stump a, the center b of the epithelium defect stump, and the thickness of granulation tissue formed in tissue 3 below a midpoint c between a and b (see FIG. 4B), and an average value was taken as the thickness of the formed granulation tissue (see FIG. 4B).

### Quantitative Reverse Transcription-Polymerase Chain Reaction (qRT-PCR)

To extract mRNA from the granulation tissue from wounds caused by burns, target sites were selectively collected from each sample. When sampling was performed, the granulation tissue was dissected in the same manner for all specimens, which were made as anatomically uniform as possible so as not to be affected by differences in sampling sites and regions. Total mRNA was extracted from tissue using Isogen II (Nippon Gene Co. LTD., Tokyo, Japan) according to the manufacturer's instructions. A 3 µg part of the mRNA was treated with deoxyribonuclease I (DNase I, Sigma-Aldrich, Inc., Tokyo, Japan) for 15 minutes at room temperature. cDNA was synthesized using 500 ng of DNase I-treated RNA using the ReverTra Ace qPCR RT Kit (made by Toyobo Co., Ltd., Osaka, Japan). Gene expression was subjected to real-time RT-PCR analysis using Brilliant SYBR Green QRT-PCR Mix (Stratagene; Agilent Technologies Japan Ltd. Japan) using an Mx3000P quantitative polymerase chain reaction (qPCR) system (Stratagene; Agilent Technologies Japan Ltd, Japan). On POD 1, POD 4, and POD 7, mRNA was extracted from each of five mice in each group, and the growth factors TGFβ130, VEGFA31, α-SMA32, bFGF33, and PDGF34, the cell migration chemokines CXCL-535 and SDF-135, CD163 which is a marker for anti-inflammatory M2 macrophages, the anti-inflammatory cytokine IL-1037, the inflammatory cytokine IL-637, tumor necrosis factor-alpha (TNF-α38), and inducible nitric oxide synthase (iNOS38) were measured in triplicate.

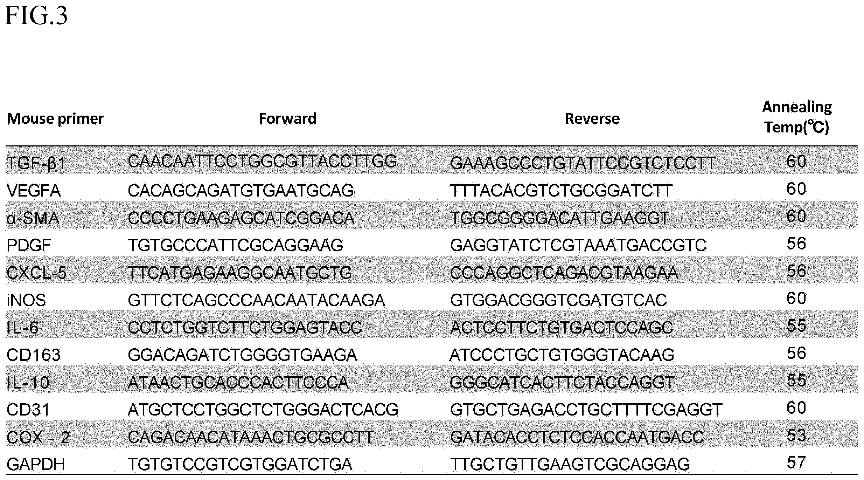

To establish that the variations in cytokines were postoperative changes, tissue was collected immediately after creating five model mice, and the mRNA expression level of each primer immediately after the operation was examined as POD 0. The expression level of each mRNA was corrected with GAPDH as an internal control, and relative comparisons were made based on the mean value of the HD-AM(-) group on POD 7. The sequence of the primers used here is depicted in FIG. 3.

### Evaluation of Thickness of Granulation Tissue at the Treatment Site of Burn

The thickness of the granulation tissue at the wound part was measured in each group (indicated by yellow arrows) and was compared on POD 1, POD 4, and POD 7 (see FIG. 5A). The HD-AM group had significantly thicker granulation tissue than the HD-AM(-) group on all of POD 1, POD 4, and POD 7 (see FIG. 5B: p < 0.05).

### Cell Infiltration into HD-AM

Cell infiltration into the HD-AM was observed for the HD-AM group (see FIG. 6). By histologically observing the amniotic membrane placed at the wound, higher inflammatory cell infiltration was confirmed for the HD-AM group at the lower part of the amniotic membrane. It is believed that this indicates the functioning of HD amniotic membrane as a scaffold for regeneration.

### Comparison of Pro-Inflammatory and Anti-Inflammatory Patterns between HD-AM group and HD-AM(-) group

FIG. 7 depicts measurements of anti-inflammatory and inflammatory cytokines, with the HD-AM group indicating an increase in expression of inflammatory cytokine mRNA followed by expression of a large amount of anti-inflammatory cytokine mRNA. This suggests that the wound healing process is clearly and smoothly promoted.

The inflammatory cytokine iNOS significantly increased in the HD-AM group on POD 4 and POD 7. The inflammatory cytokine IL-6 was transiently elevated on POD 4. The anti-inflammatory cytokine IL-10 was significantly elevated for the HD-AM group on POD 4 and POD 7. CD163, which indicates anti-inflammatory M2 macrophages, was significantly elevated in the HD-AM group on OD 7 (see FIG. 7).

CD 163, a marker for anti-inflammatory M2 macrophages that act on tissue remodeling, indicated significantly higher values for the HD-AM group on POD7 (see FIG. 7). These cytokines are useful in the early stage of wound healing, but if a high level of expression is prolonged, they are believed to be factors for scar formation, chronic wounding, and the like, indicating that HD-AM can suppress tissue damage due to scarring and prolongation of inflammation. In HD-AM treatment, a pattern with transient promotion of inflammation followed by a switch to an anti-inflammatory state is thought to be important.

Regarding anti-inflammatory cytokines, IL-10 also tended to be higher when amniotic membrane was placed. These results indicate that the application of HD amniotic membrane has the effect of promoting healing of a wound.

### Immunohistochemical Staining

Localization of CD31, which indicates neovascularization, TGFβ-1, which also contributes to growth suppression, cell differentiation, and apoptosis induction, VEGF, a vascular endothelial growth factor, and CD163, which indicates anti-inflammatory M2 macrophages, were investigated by immunohistochemical staining. On POD 7 for the HD-AM group, neovascularization was observed at the granulation site (tissue 3) in a state that extended in a direction toward tissue 1 (see FIG. 8A). At tissue 1, infiltrated cells had achieved engraftment with the HD-AM as a scaffold even on POD 7, and Iba1-positive cells (macrophages: both M1 and M2) had secreted TGF-b and VEGF (see FIG. 8B). On POD 4, VEGF was positive in tissue 2 for the HD-AM group and many cells secreting VEGF were present, and on POD7, cells that secreted VEGF were observed in cells that had HD-AM as a scaffold (see FIG. 8C). Proliferation of vascular endothelial cells caused neovascularization, which resulted in the formation of favorable granulation tissue. It was suggested that due to the effect of these cytokines, the formation of effective granulation that is rich with blood vessels was promoted in the subcutaneous tissue.

### Manufacture of HD-AM

Using the drying device depicted in FIG. 9, a raw amniotic membrane was dried with vacuum, far-infrared, and microwave devices set at the conditions given below.

The drying device in FIG. 9 is equipped, inside a processing tank 10, with a rotary table 12 on which raw amniotic membrane is placed, and a far-infrared heater 14 as a heating means. Also, to reduce the pressure inside the processing tank 10, a solenoid valve 20 and a vacuum pump 18 are connected to the processing tank 10. Also, to repressurize the processing tank 10 interior, a solenoid valve 26 and a filter 24 are connected to the processing tank 10.

The treatment tank 10 is also provided with a microwave irradiating device 30 which performs, when a pressure recovery operation has been performed from a reduced pressure state, drying while applying energy to water molecules in the amniotic membrane

Drying tank heating temperature: 50°C, FIR: 50°C, Stop valve: 37%, Maximum ultimate pressure 0.34kPa Dry running maximum ultimate pressure 0.33kPa

### Drying method

(1) Depressurization 180sec
(2) Pressure recovery 30 sec (with stop valve 37% open)
   Commence emission of microwaves
   0.1kw for 180sec (pressure recovery continues)
(3) Depressurization 180 sec
(4) Repeat (2) and (3) thereafter
(5) End drying manually by checking the ultimate pressure after 180 sec of depressurization in (3) is (0.30 to 0.35 kPa).

Process complete when atmospheric pressure is restored

### Industrial Applicability

Dried amniotic membrane (HD-AM) is manufactured by a specific drying process, that is, raw amniotic membrane that has been placed inside a processing tank is continuously heated by a far infrared heater provided inside the processing tank while performing a depressurization operation that reduces the pressure inside the processing tank and irradiation of the raw amniotic membrane with microwaves from a microwave irradiating device provided inside the processing tank to apply energy to the water molecules present inside the amniotic membrane and dry the amniotic membrane during a pressure recovery operation that slightly raises the pressure inside the depressurized processing tank toward atmospheric pressure. By repeating the above multiple times, it is possible to improve storage stability and handling of the dried amniotic membrane while preserving the cell and tissue structure. This is a treatment method which, by using dried amniotic membrane when covering a dermal defect or subcutaneous tissue of a patient with a severe burn and/or during tissue regeneration (favorable formation of granulation that accompanies neovascularization), can quickly realize a skin graft, which is essential for treating third-degree burns and the like. In addition, by using amniotic membrane to prevent infection at a wound caused by a burn, it is possible to increase the patient's survival rate and achieve therapeutic effects (that is, eliminate the risk of keloids).

## Claims

1. A restoration material to be used in treatment of burns as a covering material for burns and/or a regenerative scaffold for burns,
the restoration material comprising a dry amniotic membrane produced by performing a drying process on raw amniotic membrane that surrounds a fetus of an animal, including a human,
wherein the dry amniotic membrane has been dehydrated and dried so as to enable storage in an aseptic dry atmosphere, and
amniotic membrane produced by rehydrating the dry amniotic membrane by immersion in water or a buffer retains epithelial cells, basement membrane, and connective tissue constituting the raw amniotic membrane.

2. The restoration material according to claim 1,
wherein the burns are second-degree and/or third-degree burns.

3. Use of a restoration material for burns to be used in treatment of burns as a covering material for burns and/or a regenerative scaffold for burns,
the restoration material being a dry amniotic membrane produced by performing a drying process on raw amniotic membrane that surrounds a fetus of an animal, including a human,
wherein the dry amniotic membrane has been dehydrated and dried so as to enable storage in an aseptic dry atmosphere, and
amniotic membrane produced by rehydrating the dry amniotic membrane by immersion in water or a buffer retains epithelial cells, basement membrane, and connective tissue constituting the raw amniotic membrane.

4. The use of a restoration material for burns according to claim 3,
wherein the burns are second-degree and/or third-degree burns.

5. A method of treating burns which uses, as a covering material for burns and/or a regenerative scaffold for burns, dry amniotic membrane, which has been produced by performing a drying process on raw amniotic membrane that surrounds a fetus of an animal, including a human, wherein the dry amniotic membrane has been dehydrated and dried so as to enable storage in an aseptic dry atmosphere, and amniotic membrane produced by rehydrating the dry amniotic membrane by immersion in water or a buffer retains epithelial cells, basement membrane, and connective tissue constituting the raw amniotic membrane.

6. The method of treating burns according to claim 5,
wherein the burns are second-degree and/or third-degree burns.
